# EUROPEAN PATENT APPLICATION

(11) **EP 4 628 001 A1**
(43) Date of publication of application: **08.10.2025**
(21) Application number: 25162750.1
(22) Date of filing: 10.03.2025
(51) Int. Cl.: A61B 5/00, A61B 5/332, A61B 5/339

(54) **SYSTEM AND METHOD FOR EASY READING OF INTERVALS OF ELECTROCARDIOGRAM SIGNALS**

(30) Priority: 02.04.2024 US 202418624343
(71) Applicant: GE Precision Healthcare LLC, Waukesha, WI 53188 (US)
(72) Inventor: VENON, Medhi Olivier, Waukesha, 53188 (US); YOUNG, Brian Joseph, Waukesha, 53188 (US); OGBONNA, Benjamin Obinna, Waukesha, 53188 (US)
(74) Representative: Kilburn & Strode LLP

(57) **Abstract**

A method includes receiving an electrocardiogram signal obtained of a subject. The method also includes displaying the electrocardiogram signal over a grid on a display. The grid includes both vertical lines and horizontal lines that define a first of plurality of squares and a second plurality of smaller squares within each square of the first plurality of squares. Both the first plurality of squares and the second plurality of smaller squares represent an interval of time in a horizontal direction. Both the first plurality of squares and the second plurality of squares represent a voltage or electrical potential level in a vertical direction. The method further includes independently moving either the electrocardiogram signal relative to the grid or the grid relative to the electrocardiogram signal to align the electrocardiogram signal with an outer edge of a respective square of the first plurality of squares in response to a user input.

## Description

### BACKGROUND

The subject matter disclosed herein relates to medical monitoring systems and devices and, more particularly, to a system and a method for easy reading of intervals of electrocardiogram signals.

Electrocardiograms (ECGs) are graphic depictions of electrical activity in the heart. ECGs are produced by electrocardiographs which are available as stand-alone devices, portable devices, and/or as integrated functions in various types of multi-vital sign monitoring devices. ECGs are depicted by time (milliseconds, ms) versus voltage (microvolts, µV) and typically are represented as a waveform. The typical five important aspects, or portions, of an ECG waveform are the P wave, QRS complex (represented as the combination of the Q, R, and S waves respectively), and T wave. The less frequently seen sixth portion is a U wave. The data produced from the graphical depictions are useful in diagnosis of patients to determine what, if any, and the extent to which heart-related problems exist in a patient. For instance, ECGs are used in diagnosing cardiac arrhythmias (irregular heart rhythms), myocardial infarction (heart attacks), hyper- and hypokalemia (high or low potassium levels, respectively), blockage, ischemia (loss of oxygen due to lack of blood flow possibly from blockage), just to name a few, and may also assist in diagnosis of non-heart related ailments. Accordingly, ECGs are known and proven to be valuable tools in diagnosis heart and even non-heart-related problems with patients.

Particularly, the ECG waveforms are useful in determining whether certain conditions exist or the predisposition of such conditions occurring based on established patterns. Particularly, important information can be derived by measuring the time between certain waveforms. Commonly reviewed time intervals are those between the P wave and the beginning of the QRS interval (known as the PR interval) and the time between the QRS complex and the T wave (known as the QT interval). Other relevant data may be derived from the PR segment, the QRS complex, and the ST segment.

### BRIEF DESCRIPTION

A summary of certain embodiments disclosed herein is set forth below. It should be understood that these aspects are presented merely to provide the reader with a brief summary of these certain embodiments and that these aspects are not intended to limit the scope of this disclosure. Indeed, this disclosure may encompass a variety of aspects that may not be set forth below.

In one embodiment, a computer-implemented method for reading intervals of an electrocardiogram signal is provided. The computer-implemented method includes receiving, at a processor, an electrocardiogram signal obtained of a subject. The computer-implemented method also includes displaying, via the processor, the electrocardiogram signal over a grid on a display of a computing device. The grid includes both vertical lines and horizontal lines that define a first of plurality of squares and a second plurality of smaller squares within each square of the first plurality of squares. Both the first plurality of squares and the second plurality of smaller squares represent an interval of time in a horizontal direction, and both the first plurality of squares and the second plurality of squares represent a voltage or electrical potential level in a vertical direction. The computer-implemented method further includes independently moving, via the processor, either the electrocardiogram signal relative to the grid or the grid relative to the electrocardiogram signal to align the electrocardiogram signal with an outer edge of a respective square of the first plurality of squares in response to a user input provided via a user input device by a user.

In another embodiment, a system for reading intervals of an electrocardiogram signal is provided. The system includes a memory encoding processor-executable routines. The system also includes a processor configured to access the memory and to execute the processor-executable routines, wherein the routines, when executed by the processor, cause the processor to perform actions. The actions include receiving an electrocardiogram signal obtained of a subject. The actions also include displaying the electrocardiogram signal over a grid on a display of a computing device. The grid includes both vertical lines and horizontal lines that define a first of plurality of squares and a second plurality of smaller squares within each square of the first plurality of squares. Both the first plurality of squares and the second plurality of smaller squares represent an interval of time in a horizontal direction, and both the first plurality of squares and the second plurality of squares represent a voltage or electrical potential level in a vertical direction. The actions further include independently moving either the electrocardiogram signal relative to the grid or the grid relative to the electrocardiogram signal to align the electrocardiogram signal with an outer edge of a respective square of the first plurality of squares in response to a user input provided via a user input device by a user.

In a further embodiment, a non-transitory computer-readable medium, the computer-readable medium including processor-executable code that when executed by a processor, causes the processor to perform actions. The actions include receiving an electrocardiogram signal obtained of a subject. The actions also include displaying the electrocardiogram signal over a grid on a display of a computing device. The grid includes both vertical lines and horizontal lines that define a first of plurality of squares and a second plurality of smaller squares within each square of the first plurality of squares. Both the first plurality of squares and the second plurality of smaller squares represent an interval of time in a horizontal direction, and both the first plurality of squares and the second plurality of squares represent a voltage or electrical potential level in a vertical direction. The actions further include independently moving either the electrocardiogram signal relative to the grid or the grid relative to the electrocardiogram signal to align the electrocardiogram signal with an outer edge of a respective square of the first plurality of squares in response to a user input provided via a user input device by a user.

### BRIEF DESCRIPTION OF THE DRAWINGS

These and other features, aspects, and advantages of the present subject matter will become better understood when the following detailed description is read with reference to the accompanying drawings in which like characters represent like parts throughout the drawings, wherein:
FIG. 1 illustrates an ECG waveform having normal intervals;
FIG. 2 illustrates a schematic diagram of an electrocardiogram monitoring system, in accordance with aspects of the present disclosure;
FIG. 3 illustrates a schematic diagram of a computing device utilized for reading intervals of electrocardiogram signals, in accordance with aspects of the present disclosure;
FIG. 4 illustrates a schematic diagram of a portion of a grid utilized to measure parameters related to electrocardiogram signals, in accordance with aspects of the present disclosure;
FIG. 5 illustrates a flow diagram of a method for reading intervals of an electrocardiogram signal, in accordance with aspects of the present disclosure;
FIG. 6 illustrates a flow diagram of a method for reading intervals of an electrocardiogram signal (e.g., utilizing march out markers), in accordance with aspects of the present disclosure;
FIG. 7 illustrates an electrocardiogram signal rendered over a grid on a display of a smartphone (e.g., with the electrocardiogram signal not aligned with the grid), in accordance with aspects of the present disclosure;
FIG. 8 illustrates an electrocardiogram signal rendered over a grid on a display of a smartphone (e.g., with the electrocardiogram signal aligned with the grid), in accordance with aspects of the present disclosure;
FIG. 9 illustrates an electrocardiogram signal rendered over a grid on a display of a computing device having a larger screen (e.g., with the electrocardiogram signal not aligned with the grid), in accordance with aspects of the present disclosure;
FIG. 10 illustrates an electrocardiogram signal rendered over a grid on a display of a computing device having a larger screen (e.g., with the electrocardiogram signal aligned with the grid), in accordance with aspects of the present disclosure;
FIG. 11 illustrates an electrocardiogram signal rendered over a grid on a display of a computing device (e.g., with the march out markers not aligned with the grid), in accordance with aspects of the present disclosure;
FIG. 12 illustrates an electrocardiogram signal rendered over a grid on a display of a computing device (e.g., with the march out markers aligned with the grid), in accordance with aspects of the present disclosure; and
FIG. 13 illustrates a flow diagram of a method for reading intervals of physiological signal, in accordance with aspects of the present disclosure.

### DETAILED DESCRIPTION

One or more specific embodiments will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present subject matter, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be nonlimiting, and thus additional numerical values, ranges, and percentages are within the scope of the disclosed embodiments.

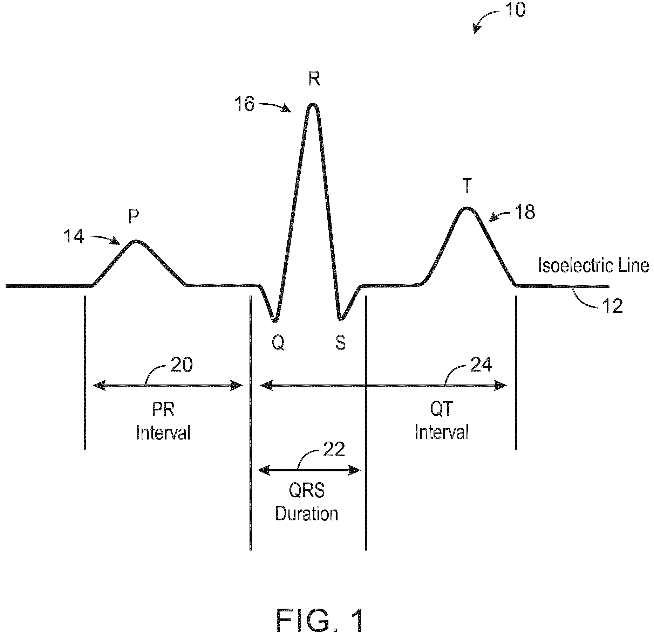

A cardiologist, or any medical professional trained to interpret electrocardiograms (ECGs or EKGs), uses the grid on an ECG printout to analyze and measure various aspects of the heart's electrical activity. The following describes how a cardiologist reads an ECG test and uses the grid to make measurements in reference to an ECG waveform 10 having normal intervals illustrated in FIG. 1.

The cardiologist starts by identifying the baseline or isoelectric line 12 on the ECG. This is the line where the electrical activity of the heart is at rest (neither positively nor negatively charged). The cardiologist then examines the waveforms on the ECG. The main components of the ECG waveform 10 include the P-wave 14, QRS complex 16, and T-wave 18. They measure the duration and amplitude (height) of these waveforms. The duration is measured in seconds (time) using the horizontal grid lines, while the amplitude is measured in millivolts (voltage) using the vertical grid lines. Various intervals are measured, including the PR interval 20 (from the beginning of the P-wave 14 to the beginning of the QRS complex 16), the QRS duration 22 (the width of the QRS complex 16), and the QT interval 24 (from the beginning of the QRS complex 16 to the end of the T-wave 18). These intervals are crucial for assessing different aspects of cardiac conduction. The grid is used to calculate the various interval without additional tools. The cardiologist zooms in to magnify some of the details. The user then drags the magnified tests to a position. However, the grid and the waveform move together. It is challenging when the interval the user intends to assess is not aligned with the grid in the background. In the case of the march out, it could be even more challenging.

The present disclosure provides systems and methods to addresses this interaction. In particular, the present disclosure provides systems and methods for reading intervals of an electrocardiogram signal that enables the action of dragging with an input device or gesture to move the ECG or the grid independently of each other, thus, allowing the user to align horizontally and/or vertically the waveform to the grid to read the number of big and small squares and to mentally calculate the interval without the use of any measurement tool. The disclosed embodiments are more efficient by allowing the adjustment of alignment compared to adjusting the caliper on the targeted lead and adjusting the left and the right edges of the caliper to determine the values.

For example, the systems and methods include receiving an electrocardiogram signal obtained of a subject. The systems and methods also include displaying the electrocardiogram signal over a grid on a display of a computing device. The grid includes both vertical lines and horizontal lines that define a first of plurality of squares and a second plurality of smaller squares within each square of the first plurality of squares. Both the first plurality of squares and the second plurality of smaller squares represent an interval of time in a horizontal direction, and both the first plurality of squares and the second plurality of squares represent a voltage or electrical potential level in a vertical direction. The systems and methods further include independently moving either the electrocardiogram signal relative to the grid or the grid relative to the electrocardiogram signal to align the electrocardiogram signal with an outer edge of a respective square of the first plurality of squares in response to a user input provided via a user input device by a user.

In certain embodiments, the systems and methods include independently moving, via the processor, the electrocardiogram signal relative to the grid to align the electrocardiogram signal with the outer edge in response to the user input. In certain embodiments, the disclosed systems and methods include independently moving, via the processor, the electrocardiogram signal relative to the grid to align the electrocardiogram signal with the outer edge in response to the user input. In certain embodiments, independent movement of either the electrocardiogram signal relative to the grid or the grid relative to the electrocardiogram signal is in the vertical direction and the outer edge includes a respective horizontal line of the horizontal lines. In certain embodiments, independent movement of either the electrocardiogram signal relative to the grid or the grid relative to the electrocardiogram signal is in the horizontal direction and the outer edge includes a respective vertical line of the vertical lines.

In certain embodiments, the systems and methods include displaying, via the processor, march out markers over the grid on the display of the computing device. In certain embodiments, the systems and methods include independently moving, via the processor, either the march out markers relative to the grid or the grid relative to the march out markers to align the march out markers with some of the vertical lines of the grid in response to additional user input provided via the user input device by the user prior to independently moving either the electrocardiogram signal relative to the grid or the grid relative to the electrocardiogram signal.

In certain embodiments, the display of the computing device is on a smartphone (i.e., in smaller form on a smaller screen). For example, a mobile application is utilized by the smartphone for manipulating the ECG signal relative to the grid. In certain embodiments, the display is on a larger screen (i.e., in larger form). For example, display is on a laptop, a tablet, or a monitor coupled to a desktop computer. For example, a desktop application is utilized by the laptop, the tablet, or the desktop computer for manipulating the ECG signal relative to the grid.

Although the techniques described below are utilized in conjunction with an electrocardiogram signal, the techniques may be utilized in other applications. For example, the disclosed techniques may be utilized with any physiological signal presented on a background pattern that measures at least one parameter in at least one direction (e.g., time).

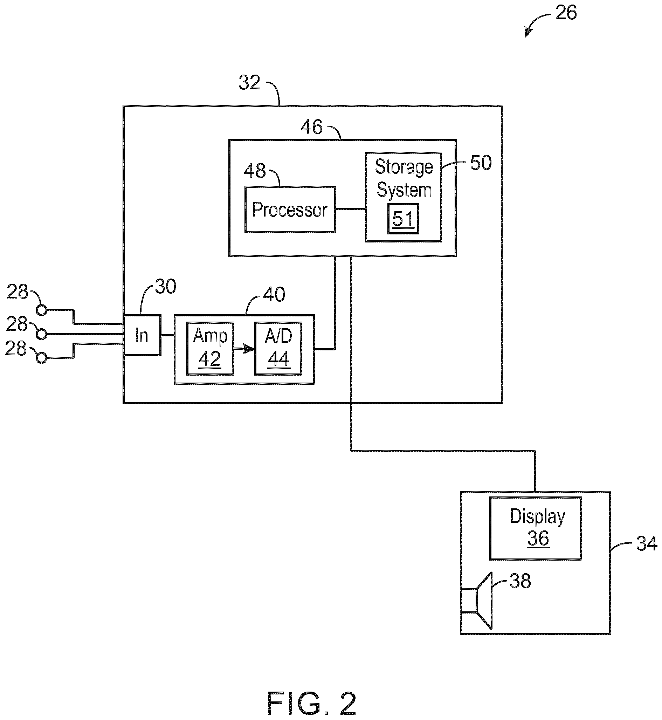

With the preceding in mind, FIG. 2 is a schematic diagram of an electrocardiogram (ECG) monitoring system 26 is illustrated. As depicted, the ECG monitoring system 26 includes three ECG electrodes 28 connected to an input port 30 of a monitor 32. While the example of FIG. 1 depicts an embodiment including three electrodes, a person of ordinary skill in the art will understand in light of this disclosure that the ECG monitoring system 26 may include any number of two or more electrodes, and that common electrode arrangements for standard diagnostic ECGs include anywhere from three to fourteen electrodes with 10 electrodes being the most commonly used electrode configuration for diagnostic ECGs. As described in more detail herein, the ECG monitoring system 26 also includes a user interface 34 connected to the monitor 32 to receive control inputs from a user, such as a clinician administering an ECG to a patient, and to provide auditory or visual outputs to the user. Accordingly, the user interface 34 includes a display 36 and a speaker 38. The user interface 34 may be a separate device that is electrically or wirelessly connected to the monitor 32, or the user interface 34 may be integrated with the monitor 32, such as within the same housing. Alternatively, the user interface 34 may include a printer that may be used as an output device to produce a printed form of the ECG dataset as the display.

The cardiac potentials recorded by the electrodes 28 are then processed by signal processing circuit 40, which includes one or more amplifiers 42 and one or more analog-to-digital converters 44. For example, the amplifier 42 may be a differential amplifier that compares potentials measured by various electrodes 28, or compares the potentials measured at each electrode to a reference input (such as ground or an active drive voltage) to derive a signal which is then utilized by the computing system 46 to generate the ECG lead signals. The output from the amplifier 42 is digitized by the analog-to-digital converter (A/D converter) 44. The A/D converter 44 may be any device or logic set capable of digitizing analog physiological signals at an appropriate sampling rate. For example, the A/D converter 44 may be an analog front end (AFE). The signal processing circuit 40 may include multiple amplifiers 42 and A/D converters 44, such as one for each electrode in the system 26. For example, a 10 electrode set is configured so that one electrode is connected to a ground reference and the remaining 9 electrodes are used as inputs to 8 amplifiers and are digitized by 8 A/D converters to generate signals from which a standard 12-lead ECG is derived.

The output of the signal processing circuit 40 is received by the computing system 46 within the monitor 32. The computing system 46 includes one or more processors 48 and storage system 50 (e.g., memory). An application 51 (e.g., mobile or desktop application) is stored within storage system 50, which is a set of software instructions executable by the processor 48 to enable the independent movement of an acquired ECG signal relative to a grid or the grid relative to the ECG signal as described below.

The processor 48 may be any type of computer processor or microprocessor capable of executing computer-executable code. The processor 48 may also include multiple processors that may perform the operations described below. In addition, the memory 50 may be any suitable articles of manufacture that can serve as media to store processor-executable code, data, or the like. These articles of manufacture may represent computer-readable media (e.g., any suitable form of memory or storage) that may store the processor-executable code used by the processor 48 to perform the presently disclosed techniques. The memory 50 may also be used to store data, consumer models, various other software applications, and the like. The memory 50 may represent non-transitory computer-readable media (e.g., any suitable form of memory or storage) that may store the processor-executable code used by the processor 48 to perform various techniques described herein. It should be noted that non-transitory merely indicates that the media is tangible and not a signal.

In particular, the processor 48 is configured to receive an electrocardiogram signal obtained of a subject. The processor 48 is also configured to display the electrocardiogram signal over a grid on a display of a computing device. The grid includes both vertical lines and horizontal lines that define a first of plurality of squares and a second plurality of smaller squares within each square of the first plurality of squares. Both the first plurality of squares and the second plurality of smaller squares represent an interval of time in a horizontal direction, and both the first plurality of squares and the second plurality of squares represent a voltage or electrical potential level in a vertical direction. The processor 48 is further configured to independently move either the electrocardiogram signal relative to the grid or the grid relative to the electrocardiogram signal to align the electrocardiogram signal with an outer edge of a respective square of the first plurality of squares in response to a user input provided via a user input device by a user.

In certain embodiments, the processor 48 is configured to display march out markers over the grid on the display of the computing device. In certain embodiments, the processor 48 is configured to independently move either the march out markers relative to the grid or the grid relative to the march out markers to align the march out markers with some of the vertical lines of the grid in response to additional user input provided via the user input device by the user prior to independently moving either the electrocardiogram signal relative to the grid or the grid relative to the electrocardiogram signal.

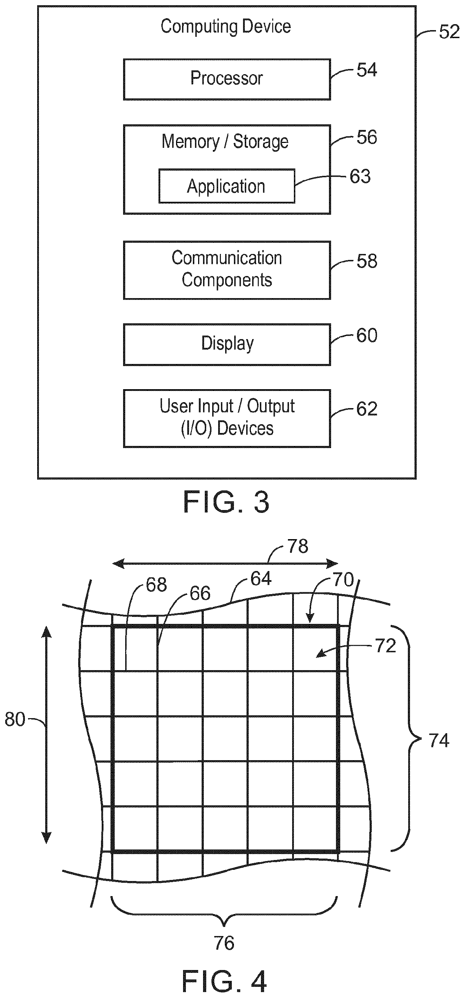

FIG. 3 illustrates a schematic diagram of a computing device 52 (e.g., user device) utilized for reading intervals of electrocardiogram signals. The computing device 52, may be implemented on one or more suitable electronic computing devices, such as a laptop, personal computer, mobile device, smartphone, tablet, wearable device, and so on. To perform one or more operations described herein, the computing device 52 may include various types of components that may assist the computing device 52 in performing the operations described below (e.g., enabling the independent movement of an acquired ECG signal relative to a grid or the grid relative to the ECG signal). For example, as shown in FIG. 3, the computing device 52 may include a processor 54, memory/storage 56, a communication component 58, a display 60, input/output (I/O) devices 62 (e.g., mouse, keyboard, microphone, touchscreen (e.g., integrated within the display 60), stylus, etc.), and the like, in accordance with embodiments described herein.

The processor 54 may be any type of computer processor or microprocessor capable of executing computer-executable code. The processes and logic flows described in this specification may be performed by one or more programmable processors executing one or more computer programs to perform functions by operating on input data and generating output. The processes and logic flows may also be performed by special purpose logic circuitry, e.g., an FPGA (field programmable gate array) or an ASIC (application specific integrated circuit). Processors suitable for the execution of a computer program include, by way of example, both general and special purpose microprocessors, and/or processor(s) of any appropriate kind of digital computer.

The memory and the storage 56 may be any suitable articles of manufacture that store processor-executable code, data, or the like. These articles of manufacture may include non-transitory computer-readable media (e.g., any suitable form of memory or storage) that may store the processor-executable code used by the processor 54 to perform the presently disclosed techniques. As used herein, applications (e.g., mobile application or desktop application) may include any suitable computer software or program that may be installed onto the computing device 52 and executed by the processor 54. It should be noted that non-transitory merely indicates that the media is tangible and not a signal. The computing device 52 may also be communicatively coupled to a network 20 that also may be communicatively coupled to an ECG monitoring system (e.g., electrocardiogram (ECG) monitoring system 26 in FIG. 2).

The computing device 52 may also include one or more user interface applications including an application 63 for enabling the independent movement of an acquired ECG signal relative to a grid or the grid relative to the ECG signal as described in greater detail below. In particular, the application 63 may cause a user interface to be displayed on the electronic display 60 of the computing device 52 that enables the display of one or more electrocardiogram signals relative to a grid. In response to user inputs (e.g., via the input/output (I/O) devices 62), the user interface may enable movement of the electrocardiogram signal relative to a grid or the grid relative to the electrocardiogram signal.

FIG. 4 illustrates a schematic diagram of a portion of a grid 64 utilized to measure parameters related to electrocardiogram signals. The grid 64 serves as a background that one or more electrocardiogram signals may be rendered over. The grid 64 enables measurement of parameters related to the electrocardiogram signals. The grid 64 consists of vertical lines 66 and horizontal lines 68. The vertical lines 66 and horizontal lines 68 define a plurality of squares 70 (e.g., large squares or blocks). The vertical lines 66 and horizontal lines 68 also define a plurality of squares 72 (e.g., smaller squares or blocks) within each square 70. Each column 74 of the square 70 includes 5 squares 72. Each row 76 of the square 70 includes 5 squares 72. Thus, each square 70 includes 25 squares 72. A horizontal axis 78 (e.g., horizontal direction) of the grid 64 records time. A vertical axis 80 (e.g., vertical direction) of the grid 64 records ECG amplitude (e.g., voltage). The vertical lines 66 denote time intervals along the horizontal axis 78. The horizontal lines 68 denote voltage levels along the vertical axis 80. Each column 74 of the square 70 represents 0.5 millivolts. Each row 76 of the square represents 0.2 seconds. Each smaller square 72 represents 0.1 millivolts. Each smaller square 72 represents 0.04 seconds.

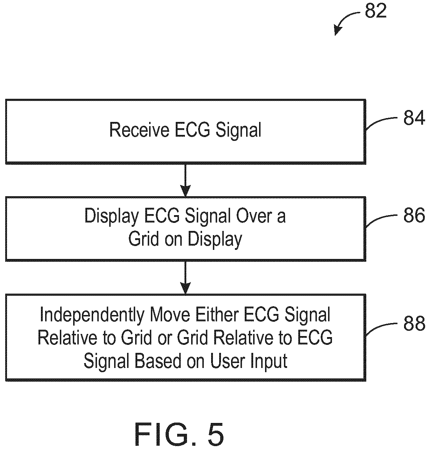

FIG. 5 illustrates a flow diagram of a method 82 for reading intervals of an electrocardiogram signal. One or more steps of the method 82 may be performed by processing circuitry of the ECG monitoring system 26 in FIG. 2 or a separate computing device 52 in FIG. 3. The method 82 may be implemented on an application (e.g., mobile application or desktop application) utilized by a computing device of the ECG monitoring system 26 or a separate computing device 52 (e.g., smartphone, table, desktop computer, laptop computer, etc.). One or more of the steps of the method 82 may be performed simultaneously or in a different order from the order depicted in FIG. 5. The method 82 may be utilized with the review of multi-lead ECG (e.g., 6 leads, 12 leads, 15 leads, etc.) such as with an ECG monitoring system. In certain embodiments, the method 82 may also be utilized with single leads (e.g., as utilized with a fitness band or smart watches).

The method 82 includes receiving an electrocardiogram (ECG) signal obtained of a subject (block 84). In certain embodiments, one or more electrocardiogram signals may be received. The method 82 also includes displaying the electrocardiogram signal over a grid on a display of a computing device (block 86). The grid includes both vertical lines and horizontal lines that define a first of plurality of squares and a second plurality of smaller squares within each square of the first plurality of squares as described in FIG. 4. Both the first plurality of squares and the second plurality of smaller squares represent an interval of time in a horizontal direction as described in FIG. 4. Both the first plurality of squares and the second plurality of squares represent a voltage or electrical potential level in a vertical direction as described in FIG. 4.

The method 82 further includes independently moving either the electrocardiogram signal relative to the grid or the grid relative to the electrocardiogram signal to align the electrocardiogram signal with an outer edge of a respective square (e.g., large square) of the first plurality of squares in response to a user input provided via a user input device by a user (block 88). In certain embodiments, the method 82 includes (e.g., only) independently moving the electrocardiogram signal relative to the grid to align the electrocardiogram signal with the outer edge in response to the user input. In certain embodiments, the method 82 includes (e.g., only) independently moving the grid relative to the electrocardiogram signal to align the electrocardiogram signal with the outer edge in response to the user input. In certain embodiments, the method 82 includes being able to independently move both the grid relative to the electrocardiogram signal and to move the electrocardiogram signal relative to the grid to align the electrocardiogram signal with the outer edge in response to the user input. In certain embodiments, a small form computing device (e.g., smartphone) may only enable moving the electrocardiogram signal relative to the grid to align the electrocardiogram signal with the outer edge in response to the user input. In certain embodiments, a computing device with a larger display (e.g., laptop or desktop computer) enables either moving the electrocardiogram signal relative to the grid to align the electrocardiogram signal with the outer edge in response to the user input or moving the grid relative to the electrocardiogram signal to the grid to align the electrocardiogram signal with the outer edge in response to the user input.

In certain embodiments, the outer edge of the large square is the outermost vertical line (e.g., on left side or right side) of the large square. Thus, independent movement of either the electrocardiogram signal relative to the grid or the grid relative to the electrocardiogram signal is in the vertical direction to align the electrocardiogram signal with the outer edge in response to the user input. In certain embodiments, the outer edge of the large square is the outermost horizontal line (e.g., on top side or bottom side) of the large square. Thus, independent movement of either the electrocardiogram signal relative to the grid or the grid relative to the electrocardiogram signal is in the horizontal direction to align the electrocardiogram signal with the outer edge in response to the user input.

In certain embodiments, the independent movement of either the electrocardiogram signal relative to the grid or the grid relative to the electrocardiogram signal is to align the electrocardiogram signal with a line disposed within an outer perimeter of a respective square (e.g., large square) of the first plurality of squares in response to a user input provided via a user input device by a user. For example, the independent movement of either the electrocardiogram signal relative to the grid or the grid relative to the electrocardiogram signal is to align the electrocardiogram signal with a vertical line within a line disposed within an outer perimeter of a respective square (e.g., large square) of the first plurality of squares in response to a user input provided via a user input device by a user. For example, the independent movement of either the electrocardiogram signal relative to the grid or the grid relative to the electrocardiogram signal is to align the electrocardiogram signal with a horizontal line within a line disposed within an outer perimeter of a respective square (e.g., large square) of the first plurality of squares in response to a user input provided via a user input device by a user.

The user input provided by the user to independently move either the electrocardiogram signal relative to the grid or the grid relative to the electrocardiogram signal may occur via a variety of different mechanisms. In certain embodiments, the user may utilize a touchscreen to independently move (e.g., drag) either the electrocardiogram signal relative to the grid or the grid relative to the electrocardiogram signal. In certain embodiments, the user may utilize a mouse to independently move (e.g., drag) either the electrocardiogram signal relative to the grid or the grid relative to the electrocardiogram signal. In certain embodiments, the user may utilize a keyboard to independently move (e.g., drag) either the electrocardiogram signal relative to the grid or the grid relative to the electrocardiogram signal. In certain embodiments, the user may utilize a voice commands (e.g., via a microphone) to independently move (e.g., drag) either the electrocardiogram signal relative to the grid or the grid relative to the electrocardiogram signal.

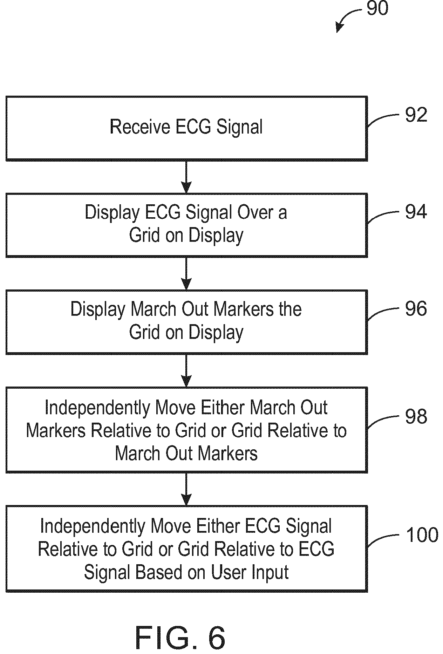

FIG. 6 illustrates a flow diagram of a method 90 for reading intervals of an electrocardiogram signal (e.g., utilizing march out markers). One or more steps of the method 90 may be performed by processing circuitry of the ECG monitoring system 26 in FIG. 2 or a separate computing device 52 in FIG. 3. The method 90 may be implemented on an application (e.g., mobile application or desktop application) utilized by a computing device of the ECG monitoring system 26 or a separate computing device 52 (e.g., smartphone, table, desktop computer, laptop computer, etc.). One or more of the steps of the method 90 may be performed simultaneously or in a different order from the order depicted in FIG. 6. The method 90 may be utilized with the review of multi-lead ECG (e.g., 6 leads, 12 leads, 15 leads, etc.) such as with an ECG monitoring system. In certain embodiments, the method 90 may also be utilized with single leads (e.g., as utilized with a fitness band or smart watches).

The method 90 includes receiving an electrocardiogram (ECG) signal obtained of a subject (block 92). In certain embodiments, one or more electrocardiogram signals may be received. The method 90 also includes displaying the electrocardiogram signal over a grid on a display of a computing device (block 94). The grid includes both vertical lines and horizontal lines that define a first of plurality of squares and a second plurality of smaller squares within each square of the first plurality of squares as described in FIG. 4. Both the first plurality of squares and the second plurality of smaller squares represent an interval of time in a horizontal direction as described in FIG. 4. Both the first plurality of squares and the second plurality of squares represent a voltage or electrical potential level in a vertical direction as described in FIG. 4.

The method 90 further includes displaying march out markers over the grid on the display of the computing device (block 96). March out markers represent a given duration interval that can be repeated across the electrocardiogram signal. The method 90 even further includes independently moving either the march out markers relative to the grid or the grid relative to the march out markers to align the march out markers with some of the vertical lines of the grid in response to additional user input provided via the user input device by the user prior to independently moving either the electrocardiogram signal relative to the grid or the grid relative to the electrocardiogram signal (block 98). In certain embodiments, the march out markers are (e.g., only) independently moved relative to the grid. In certain embodiments, the grid is (e.g., only) independently moved relative to the march out markers.

The user input provided by the user to independently move either the march out markers relative to the grid or the grid relative to the march out markers may occur via a variety of different mechanisms. In certain embodiments, the user may utilize a touchscreen to independently move (e.g., drag) either the march out markers relative to the grid or the grid relative to the march out markers. In certain embodiments, the user may utilize a mouse to independently move (e.g., drag) either the march out markers relative to the grid or the grid relative to the march out markers. In certain embodiments, the user may utilize a keyboard to independently move (e.g., drag) either the march out markers relative to the grid or the grid relative to the march out markers. In certain embodiments, the user may utilize a voice commands (e.g., via a microphone) to independently move (e.g., drag) either the march out markers relative to the grid or the grid relative to the march out markers.

The method 90 further includes independently moving either the electrocardiogram signal relative to the grid or the grid relative to the electrocardiogram signal to align the electrocardiogram signal with an outer edge of a respective square (e.g., large square) of the first plurality of squares in response to a user input provided via a user input device by a user (block 100). In certain embodiments, the method 90 includes (e.g., only) independently moving the electrocardiogram signal relative to the grid to align the electrocardiogram signal with the outer edge in response to the user input. In certain embodiments, the method 90 includes (e.g., only) independently moving the grid relative to the electrocardiogram signal to align the electrocardiogram signal with the outer edge in response to the user input. In certain embodiments, the method 90 includes being able to independently move both the grid relative to the electrocardiogram signal and to move the electrocardiogram signal relative to the grid to align the electrocardiogram signal with the outer edge in response to the user input. In certain embodiments, a small form computing device (e.g., smartphone) may only enable moving the electrocardiogram signal relative to the grid to align the electrocardiogram signal with the outer edge in response to the user input. In certain embodiments, a computing device with a larger display (e.g., laptop or desktop computer) enables either moving the electrocardiogram signal relative to the grid to align the electrocardiogram signal with the outer edge in response to the user input or moving the grid relative to the electrocardiogram signal to the grid to align the electrocardiogram signal with the outer edge in response to the user input.

In certain embodiments, the outer edge of the large square is the outermost vertical line (e.g., on left side or right side) of the large square. Thus, independent movement of either the electrocardiogram signal relative to the grid or the grid relative to the electrocardiogram signal is in the vertical direction to align the electrocardiogram signal with the outer edge in response to the user input. In certain embodiments, the outer edge of the large square is the outermost horizontal line (e.g., on top side or bottom side) of the large square. Thus, independent movement of either the electrocardiogram signal relative to the grid or the grid relative to the electrocardiogram signal is in the horizontal direction to align the electrocardiogram signal with the outer edge in response to the user input.

In certain embodiments, the independent movement of either the electrocardiogram signal relative to the grid or the grid relative to the electrocardiogram signal is to align the electrocardiogram signal with a line disposed within an outer perimeter of a respective square (e.g., large square) of the first plurality of squares in response to a user input provided via a user input device by a user. For example, the independent movement of either the electrocardiogram signal relative to the grid or the grid relative to the electrocardiogram signal is to align the electrocardiogram signal with a vertical line within a line disposed within an outer perimeter of a respective square (e.g., large square) of the first plurality of squares in response to a user input provided via a user input device by a user. For example, the independent movement of either the electrocardiogram signal relative to the grid or the grid relative to the electrocardiogram signal is to align the electrocardiogram signal with a horizontal line within a line disposed within an outer perimeter of a respective square (e.g., large square) of the first plurality of squares in response to a user input provided via a user input device by a user.

The user input provided by the user to independently move either the electrocardiogram signal relative to the grid or the grid relative to the electrocardiogram signal may occur via a variety of different mechanisms. In certain embodiments, the user may utilize a touchscreen to independently move (e.g., drag) either the electrocardiogram signal relative to the grid or the grid relative to the electrocardiogram signal. In certain embodiments, the user may utilize a mouse to independently move (e.g., drag) either the electrocardiogram signal relative to the grid or the grid relative to the electrocardiogram signal. In certain embodiments, the user may utilize a keyboard to independently move (e.g., drag) either the electrocardiogram signal relative to the grid or the grid relative to the electrocardiogram signal. In certain embodiments, the user may utilize a voice commands (e.g., via a microphone) to independently move (e.g., drag) either the electrocardiogram signal relative to the grid or the grid relative to the electrocardiogram signal.

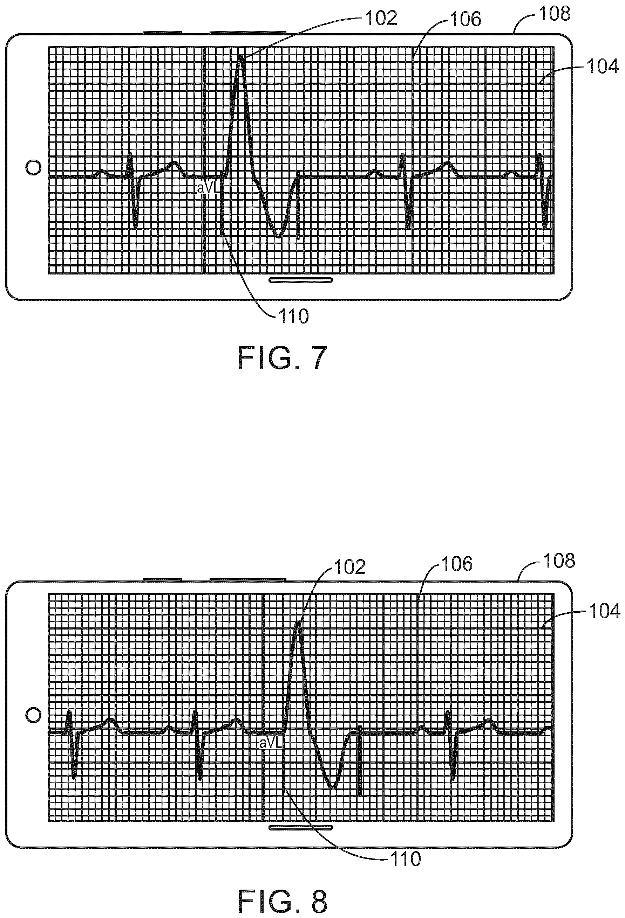

FIGS. 7 and 8 illustrate an electrocardiogram signal 102 rendered over a grid 104 on a display 106 of a smartphone 108. Calipers 110 denote an entire electrocardiogram complex within the electrocardiogram signal. As depicted in FIG. 7, the electrocardiogram signal 102 is not aligned with an outer edge (e.g., outermost vertical line) of a big square on the grid 104. As depicted in FIG. 8, the electrocardiogram signal 102 is aligned with an outer edge (e.g., outermost vertical line) of a big square on the grid 104. The electrocardiogram signal 102 was independently moved (e.g., dragged) relative to the grid 104 to align the electrocardiogram signal 102 with the outer edge of the big square on the grid 104 in response to user input provided via a user input device on the smartphone 108. In certain embodiments, the grid 104 may be independently moved (e.g., dragged) relative to the electrocardiogram signal 102 in response to user input provided via a user input device on the smartphone 108. In certain embodiments, the user input may be provided by touching the touchscreen (incorporated in the display 106) of the smartphone 108. In certain embodiments, the user input may be provided via voice commands provided via a microphone on the smartphone 108. A mobile application on the smartphone 108 enables this independent movement between the electrocardiogram signal 102 and the grid 104.

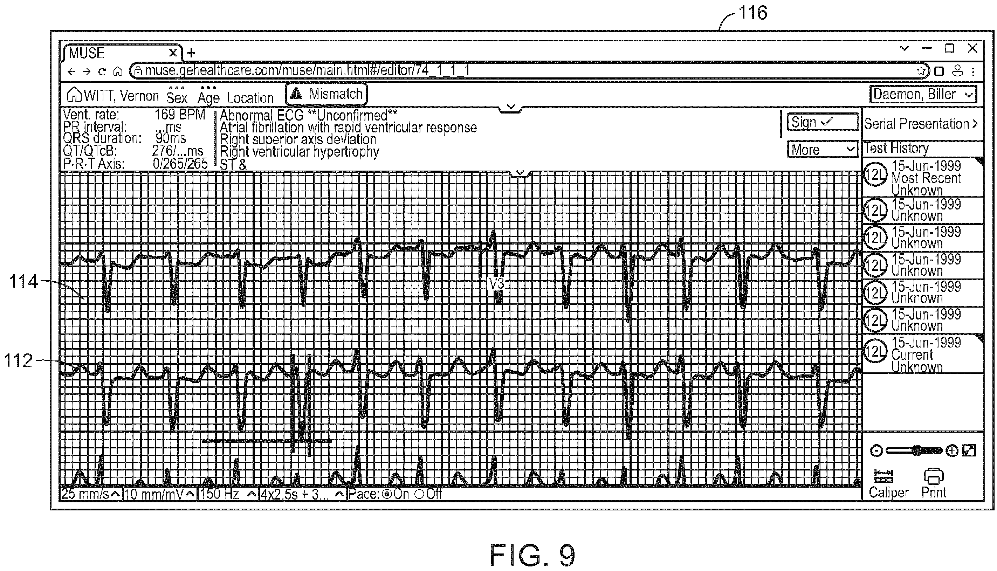

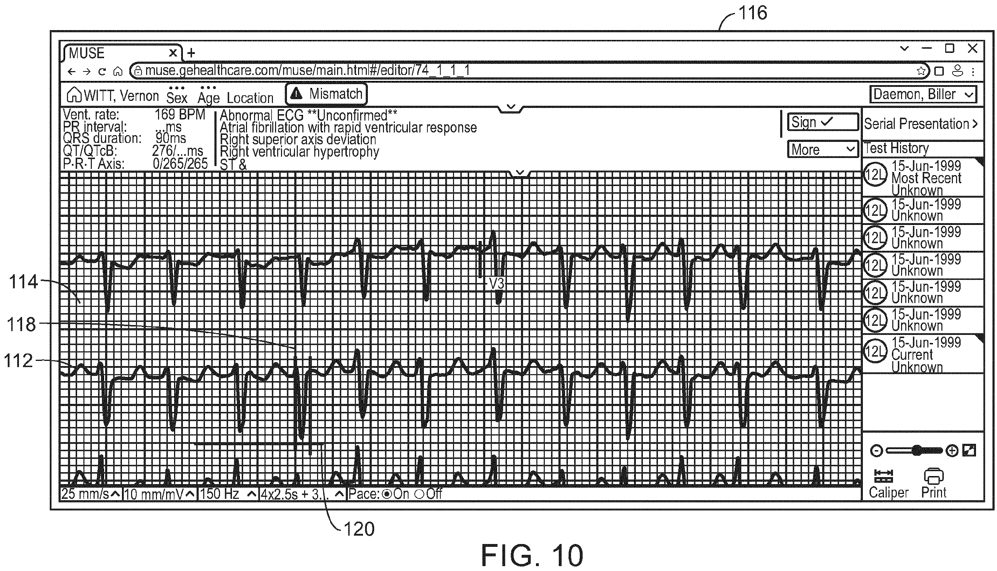

FIGS. 9 and 10 illustrate an electrocardiogram signal 112 rendered over a grid 114 on a display 116 of a larger computing device (i.e., larger than a smartphone). The display 116 may be part of a tablet, a laptop computer, or a monitor of a laptop computer. As depicted in FIG. 9, the electrocardiogram signal 112 is not aligned with an outer edge (e.g., outermost vertical line) of a big square on the grid 114. As depicted in FIG. 10, the electrocardiogram signal 112 is aligned with an outer edge (e.g., outermost vertical line) of a big square on the grid 114. In particular, the electrocardiogram signal 112 was aligned with both a vertical line 118 and a horizontal line 120 of a big square. The electrocardiogram signal 112 was independently moved (e.g., dragged) relative to the grid 114 or the grid 114 was independently moved (e.g., dragged) relative to the electrocardiogram signal 112 to align the electrocardiogram signal 112 with the outer edge of the big square on the grid 114 in response to user input provided via a user input device of the larger computing device. In certain embodiments, the user input may be provided by touching the touchscreen (incorporated in the display 116) of the larger computing device. In certain embodiments, the user input may be provided via voice commands provided via a microphone of the larger computing device. A desktop application on the larger computing device enables this independent movement between the electrocardiogram signal 112 and the grid 114.

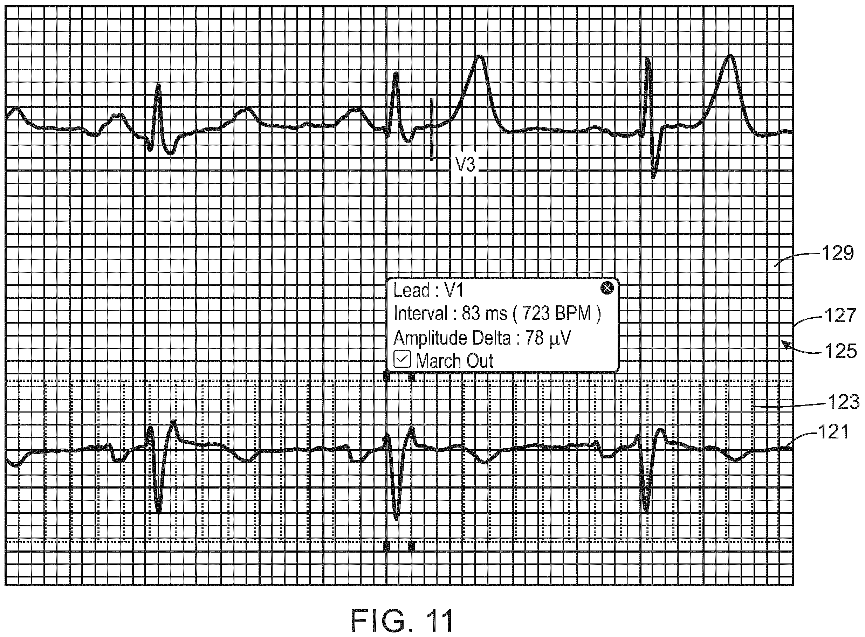

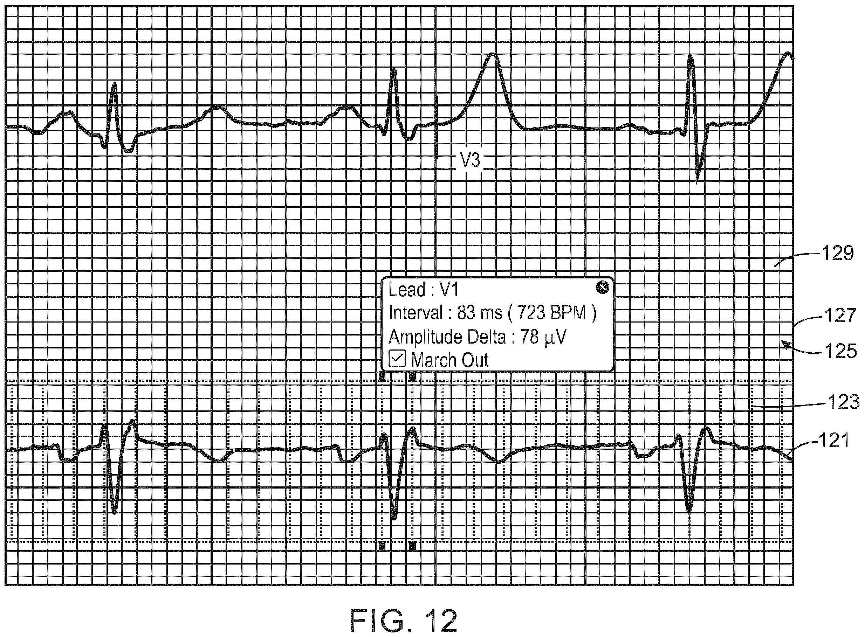

FIGS. 11 and 12 illustrate an electrocardiogram signal 121 and march out markers 123 rendered over a grid 125 on a display 127 of a computing device. The display 127 may be part of a smartphone, a tablet, a laptop computer, or a monitor of a laptop computer. As depicted in FIG. 11, the march out markers 123 are not aligned with vertical lines 129 on the grid 125. As depicted in FIG. 12, the march out markers 123 are aligned with the vertical lines 129 on the grid 125. The march out markers 123 were independently moved (e.g., dragged) together relative to the grid 125 or the grid 125 was independently moved (e.g., dragged) relative to the march out markers 123 to align the march out markers 123 with the vertical lines 129 on the grid 125 in response to user input provided via a user input device of the computing device. In certain embodiments, the user input may be provided by touching the touchscreen (incorporated in the display 127) of the computing device. In certain embodiments, the user input may be provided via voice commands provided via a microphone of the computing device.

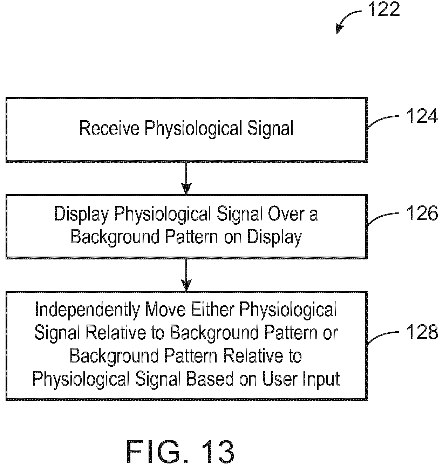

FIG. 13 illustrates a flow diagram of a method 122 for reading intervals of an physiological signal. One or more steps of the method 122 may be performed by processing circuitry of a monitoring system for one or more physiological parameters or a separate computing device 52 in FIG. 3. The method 122 may be implemented on an application (e.g., mobile application or desktop application) utilized by a computing device of the ECG monitoring system 26 or a separate computing device 52 (e.g., smartphone, table, desktop computer, laptop computer, etc.). One or more of the steps of the method 122 may be performed simultaneously or in a different order from the order depicted in FIG. 13.

The method 122 includes receiving physiological signal obtained of a subject (block 124). In certain embodiments, one or more physiological signals may be received. The method 122 also includes displaying the physiological signal over a background pattern on a display of a computing device (block 126). The background pattern includes vertical lines and/or horizontal lines that define intervals or levels for one or more parameters (e.g., time, amplitude, levels, etc.) related to the background pattern.

The method 122 further includes independently moving either the physiological signal relative to the background pattern or the background pattern relative to the physiological signal to align the physiological signal with a vertical line or a horizontal of the background pattern in response to a user input provided via a user input device by a user (block 128). In certain embodiments, the method 122 includes (e.g., only) independently moving the physiological signal relative to the background pattern to align the physiological signal with the vertical line or the horizontal line in response to the user input. In certain embodiments, the method 122 includes (e.g., only) independently moving the background pattern relative to the physiological signal to align the electrocardiogram signal with the vertical line or the horizontal line in response to the user input. In certain embodiments, the method 122 includes being able to independently move both the background pattern relative to the physiological signal and to move the physiological signal relative to the background pattern to align the physiological signal with the vertical line or the horizontal line in response to the user input. In certain embodiments, a small form computing device (e.g., smartphone) may only enable moving the physiological signal relative to the background pattern to align the physiological signal with the vertical line or the horizontal line in response to the user input. In certain embodiments, a computing device with a larger display (e.g., laptop or desktop computer) enables either moving the physiological signal relative to the background pattern to align the physiological signal with the vertical line or the horizontal line in response to the user input or moving the background pattern relative to the physiological signal to the background pattern to align the physiological signal with the vertical line or the horizontal line in response to the user input.

In certain embodiments, independent movement of either the physiological signal relative to the background pattern or the background pattern relative to the physiological signal is in the vertical direction to align the physiological signal with a vertical line in response to the user input. In certain embodiments, independent movement of either the physiological signal relative to the physiological or the physiological relative to the physiological signal is in the horizontal direction to align the physiological signal with the horizontal line in response to the user input.

The user input provided by the user to independently move either the physiological signal relative to the background pattern or the background pattern relative to the physiological signal may occur via a variety of different mechanisms. In certain embodiments, the user may utilize a touchscreen to independently move (e.g., drag) either the physiological signal relative to the background pattern or the background pattern relative to the physiological signal. In certain embodiments, the user may utilize a mouse to independently move (e.g., drag) either the physiological signal relative to the background pattern or the background pattern relative to the physiological signal. In certain embodiments, the user may utilize a keyboard to independently move (e.g., drag) either the physiological signal relative to the background pattern or the background pattern relative to the physiological signal. In certain embodiments, the user may utilize a voice commands (e.g., via a microphone) to independently move (e.g., drag) either the physiological signal relative to the background pattern or the background pattern relative to the physiological signal.

Technical effects of the disclosed subject matter include providing for the decoupling of the dragging of an electrocardiogram waveform with respect to a background grid-like pattern. Technical effects of the disclosed subject matter include reducing or eliminating the use of a calibration too. Technical effects of the disclosed subject matter include streamlining and simplifying interpretation of the electrocardiogram waveform especially for a small form factor device (e.g., smartphone). Technical effects of the disclosed subject matter include improving measurement accuracy of the electrocardiogram waveform.

The techniques presented and claimed herein are referenced and applied to material objects and concrete examples of a practical nature that demonstrably improve the present technical field and, as such, are not abstract, intangible or purely theoretical. Further, if any claims appended to the end of this specification contain one or more elements designated as "means for [perform]ing [a function]..." or "step for [perform]ing [a function]...", it is intended that such elements are to be interpreted under 35 U.S.C. 112(f). However, for any claims containing elements designated in any other manner, it is intended that such elements are not to be interpreted under 35 U.S.C. 112(f).

This written description uses examples to disclose the present subject matter, including the best mode, and also to enable any person skilled in the art to practice the subject matter, including making and using any devices or systems and performing any incorporated methods. The patentable scope of the subject matter is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A computer-implemented method for reading intervals of an electrocardiogram signal, comprising:
receiving, at a processor, an electrocardiogram signal obtained of a subject;
displaying, via the processor, the electrocardiogram signal over a grid on a display of a computing device, wherein the grid comprises both vertical lines and horizontal lines that define a first of plurality of squares and a second plurality of smaller squares within each square of the first plurality of squares, wherein both the first plurality of squares and the second plurality of smaller squares represent an interval of time in a horizontal direction, and both the first plurality of squares and the second plurality of squares represent a voltage or electrical potential level in a vertical direction; and
independently moving, via the processor, either the electrocardiogram signal relative to the grid or the grid relative to the electrocardiogram signal to align the electrocardiogram signal with an outer edge of a respective square of the first plurality of squares in response to a user input provided via a user input device by a user.

2. The computer-implemented method of claim 1, comprising independently moving, via the processor, the electrocardiogram signal relative to the grid to align the electrocardiogram signal with the outer edge in response to the user input.

3. The computer-implemented method of claim 1, comprising independently moving, via the processor, the grid relative to the electrocardiogram signal to align the electrocardiogram signal with the outer edge in response to the user input.

4. The computer-implemented method of claim 1, wherein independent movement of either the electrocardiogram signal relative to the grid or the grid relative to the electrocardiogram signal is in the vertical direction and the outer edge comprises a respective horizontal line of the horizontal lines.

5. The computer-implemented method of claim 1, wherein independent movement of either the electrocardiogram signal relative to the grid or the grid relative to the electrocardiogram signal is in the horizontal direction and the outer edge comprises a respective vertical line of the vertical lines.

6. The computer-implemented method of claim 1, further comprising:
displaying, via the processor, march out markers over the grid on the display of the computing device; and
independently moving, via the processor, either the march out markers relative to the grid or the grid relative to the march out markers to align the march out markers with some of the vertical lines of the grid in response to additional user input provided via the user input device by the user prior to independently moving either the electrocardiogram signal relative to the grid or the grid relative to the electrocardiogram signal.

7. The computer-implemented method of claim 1, wherein the display is on a smartphone.

8. The computer-implemented method of claim 1, wherein the display is on a laptop computer, a tablet, or a monitor coupled to a desktop computer.

9. A system for reading intervals of an electrocardiogram signal, comprising:
a memory encoding processor-executable routines; and
a processor configured to access the memory and to execute the processor-executable routines, wherein the processor-executable routines, when executed by the processor, cause the processor to:
receive an electrocardiogram signal obtained of a subject;
display the electrocardiogram signal over a grid on a display of a computing device, wherein the grid comprises both vertical lines and horizontal lines that define a first of plurality of squares and a second plurality of smaller squares within each square of the first plurality of squares, wherein both the first plurality of squares and the second plurality of smaller squares represent an interval of time in a horizontal direction, and both the first plurality of squares and the second plurality of squares represent a voltage or electrical potential level in a vertical direction; and
independently moving independently move either the electrocardiogram signal relative to the grid or the grid relative to the electrocardiogram signal to align the electrocardiogram signal with an outer edge of a respective square of the first plurality of squares in response to a user input provided via a user input device by a user.

10. The system of claim 9, wherein the processor-executable routines, when executed by the processor, cause the processor to independently move the electrocardiogram signal relative to the grid to align the electrocardiogram signal with the outer edge in response to the user input.

11. The system of claim 9, wherein the processor-executable routines, when executed by the processor, cause the processor to independently move the grid relative to the electrocardiogram signal to align the electrocardiogram signal with the outer edge in response to the user input.

12. The system of claim 9, wherein independent movement of either the electrocardiogram signal relative to the grid or the grid relative to the electrocardiogram signal is in the vertical direction and the outer edge comprises a respective horizontal line of the horizontal lines.

13. The system of claim 9, wherein independent movement of either the electrocardiogram signal relative to the grid or the grid relative to the electrocardiogram signal is in the horizontal direction and the outer edge comprises a respective vertical line of the vertical lines.

14. The system of claim 9, wherein the processor-executable routines, when executed by the processor, further cause the processor to
display march out markers over the grid on the display of the computing device; and
independently move either the march out markers relative to the grid or the grid relative to the march out markers to align the march out markers with some of the vertical lines of the grid in response to additional user input provided via the user input device by the user prior to independently moving either the electrocardiogram signal relative to the grid or the grid relative to the electrocardiogram signal.

15. The system of claim 9, wherein the display is on a smartphone, a laptop computer, a tablet, or a monitor coupled to a desktop computer.
